# EUROPÄISCHE PATENTSCHRIFT

(11) **EP 0 818 435 B1**
(45) Veröffentlichungstag und Bekanntmachung des Hinweises auf die Patenterteilung: **14.11.2001**
(21) Anmeldenummer: 97110471.6
(22) Anmeldetag: 26.06.1997
(51) Int. Cl.: C07C 63/70, C07C 51/38

(54) **Verfahren zur Decarboxylierung halogenierter aromatischer Carbonsäuren**
Process for the decarboxylation of aromatic carboxylic acids
Procédé de décarboxylation d'acides carboxyliques aromatiques

(30) Priorität: 09.07.1996 DE 19627411
(43) Veröffentlichungstag der Anmeldung: 14.01.1998
(73) Patentinhaber: BAYER AG, 51368 Leverkusen (DE)
(72) Erfinder: Antons, Stefan, Dr., 51373 Leverkusen (DE); Steffan, Guido, Dr., 51519 Odenthal (DE); Fiege, Helmut, Dr., 51373 Leverkusen (DE)

(56) Entgegenhaltungen:
- EP-A- 0 741 122
- EP-B- 0 194 671
- PATENT ABSTRACTS OF JAPAN vol. 095, no. 004, 31.Mai 1995 & JP 07 017904 A (ASAHI GLASS CO LTD), 20.Januar 1995,

## Beschreibung

Die vorliegende Erfindung betrifft ein besonders einfaches Verfahren zur Decarboxylierung von 2,3,5,6-Tetrafluorterephthalsäure unter Bildung von 2,3,5,6-Tetrafluorbenzoesäure.

Es sind bereits einige Verfahren zur Decarboxylierung von halogenierten aromatischen Carbonsäuren bekannt geworden. So wird in der GB-OS 2 122 190 die Decarboxylierung von 2,3,5,6-Tetrafluorterephthalsäure in polaren aprotischen Lösungsmitteln beschrieben. Die Reaktion verläuft jedoch unselektiv und liefert in der Hauptsache das vollständig decarboxylierte Produkt 1,2,4,5-Tetrafluorbenzol, ohne dass Tetrafluorbenzoesäure isoliert werden kann.

Gemäß der JP 64-25 737 wird die Decarboxylierung halogenierter aromatischer Carbonsäuren in Gegenwart von tertiären Aminen durchgeführt. Dies erfordert jedoch eine aufwendige Aufarbeitung, bei der die Reaktionsmischung erst mit Natronlauge behandelt, dann das tertiäre Amin abgetrennt und zurückgeführt und schließlich das Produkt durch Ansäuern isoliert wird.

In der EP-OS 218 111 wird vorgeschlagen diese Decarboxylierung in Gegenwart von tertiären Aminen zusätzlich in Gegenwart eines polaren aprotischen Lösungsmittels durchzuführen. Das Verfahren wird dadurch noch komplizierter.

In der JP 93-190 758 ist die Herstellung von 3,4,5,6-Tetrafluorbenzoesäure durch Erhitzen einer wässrigen Lösung von 3,4,5,6-Tetrafluorisophthalsäure auf 190°C beschrieben (siehe Beispiel 7). In Beispiel 8 wird diese Reaktion in Tri-(n-butylamin) beschrieben und eine bessere Ausbeute bei niedrigerer Reaktionstemperatur erhalten. Es bestätigt sich damit der schon nach dem anderen Stand der Technik bestehenden Eindruck, dass Basenzusätze besonders vorteilhaft sind.

Die JP 61-41 130 beschreibt schließlich die vollständige Decarboxylierung von 3,4,5,6-Tetrafluorphthalsäure in wässrigem Medium zu 1,2,3,4-Tetrafluorbenzol. Die Herstellung von 2,3,5,6-Tetrafluorbenzoesäure wird nicht beschrieben.

Die prioritätsältere, nicht veröffentlichte EP-A 741 122 beschreibt ein Verfahren zur Herstellung aromatischer Verbindungen durch Decarboxylierung aromatischer Carbonsäuren, bei dem man die wässrige Lösung mit einem in Wasser nicht löslichen, unter den Reaktionsbedingungen inerten Amin versetzt.

Die EP-A 194 671 betrifft die Herstellung von 2,3,4,5-Tetrafluorbenzoesäure aus 3,4,5,6-Tetrafluorphthalsäure. Beim Ausgangsprodukt befinden sich die beiden COOH-Gruppen also in o-Stellung zueinander.

Insbesondere im Hinblick auf die JP 61-43 130 erscheint es schwierig, die Decarboxylierung ohne Basenzusatz so durchzuführen, dass selektiv nur eine Carboxylgruppe abgebaut wird. Außerdem erscheint es als unwahrscheinlich, dass man die Decarboxylierung in wässrig-saurem Medium durchführen kann.

Es wurde nun ein Verfahren zur Decarboxylierung von 2,3,5,6-Tetrafluorterephthalsäure unter Bildung von 2,3,5,6-Tetrafluorbenzoesäure gefunden, das dadurch gekennzeichnet ist, dass man 2,3,5,6-Tetrafluorterephthalsäure zusammen mit Wasser auf Temperaturen von 80 bis 180°C oder zusammen mit Wasser und einer weiteren Säure auf Temperaturen über 80°C erhitzt.

Die 2,3,5,6-Tetrafluorterephthalsäure muss nicht in reiner Form eingesetzt werden. Man kann sie auch in wasserhaltiger Form, gegebenenfalls auch in noch weitere Säuren enthaltender Form einsetzen. Da man 2,3,5,6-Tetrafluorterephthalsäure häufig durch saure Verseifung des entsprechenden Nitrils herstellt, ist es ein großer Vorteil des erfindungsgemäßen Verfahrens, dass man direkt Reaktionsgemische aus der sauren Verseifung des Nitrils einsetzen kann. Es sind dann keine Maßnahmen zur Reinigung oder Aufarbeitung solcher Reaktionsgemische aus der sauren Nitrilverseifung notwendig.

Es kann beispielsweise 2,3,5,6-Tetrafluorterephthalsäure eingesetzt werden, die bis zu 90 Gew.-% Wasser und gegebenenfalls bis zu 60 Gew.-% weitere Säuren enthält, wobei der Gesamtgehalt von Wasser und weiterer Säure 95 Gew.-% nicht übersteigt. Vorzugsweise enthält sie 2 bis 25 Gew.-% Wasser und gegebenenfalls 1 bis 20 Gew.-% weitere Säuren. Bei den weiteren Säuren kann es sich z.B. um Schwefelsäure, Salzsäure, Phosphorsäure und/oder eine C₂- bis C₁₀-Carbonsäure, etwa Essigsäure, handeln. Bevorzugt ist Schwefelsäure, da die oben angegebene saure Verseifung des Nitrils üblicherweise mit Schwefelsäure durchgeführt wird. Selbstverständlich kann man auch 2,3,5,6-Tetrafluorterephthalsäure einsetzen, die im Rahmen der technischen Möglichkeiten frei von Wasser und/oder weiteren Säuren ist.

Das erfindungsgemäße Verfahren kann z.B. in Gegenwart der 0,5- bis 15-fachen Gewichtsmenge Wasser, bezogen auf die eingesetzte 2,3,5,6-Tetrafluorterephthalsäure durchgeführt werden. Vorzugsweise beträgt diese Menge das 0,8- bis 6-fache. Sofern die eingesetzte 2,3,5,6-Tetrafluorterephthalsäure nicht genügend Wasser enthält, ist dieses separat zuzusetzen. Weiterhin kann man das erfindungsgemäße Verfahren z.B. in Gegenwart der bis zu 15-fachen Gewichtsmenge einer oder mehrerer weiterer Säuren, bezogen auf die eingesetzte 2,3,5,6-Tetrafluorterephthalsäure durchführen. Vorzugsweise beträgt diese Menge das 0,01- bis 5-fache. Sofern die 2,3,5,6-Tetrafluorterephthalsäure nicht die gewünschte Menge weiterer Säure enthält, ist diese separat zuzusetzen.

Die 2,3,5,6-Tetrafluorterephthalsäure erhitzt man zusammen mit Wasser, vorzugsweise auf eine Temperatur von 90 bis 170°C und zusammen mit Wasser und weiterer Säure auf beispielsweise 80 bis 200°C, vorzugsweise 90 bis 180°C. Wenn die Reaktionstemperatur bei Normaldruck über der Siedetemperatur des Reaktionsgemisches liegt, ist es erforderlich, die Decarboxylierung in einem druckfesten Reaktor, z.B. einem Autoklaven, durchzuführen.

Die Reaktionszeiten können in einem weiten Bereich variiert werden und z.B. im Bereich von 1 bis 60 Stunden liegen. Im allgemeinen kann bei höheren Reaktionstemperaturen mit kürzeren Reaktionszeiten gearbeitet werden, als bei tieferen Reaktionstemperaturen. Tiefere Temperaturen, z.B. solche von 80 bis 150°C, sind dann vorteilhaft, wenn große Wassermengen und kleinere Mengen an oder keine weiteren Säuren eingesetzt werden.

Es sind wesentliche Merkmale des erfindungsgemäßen Verfahrens, dass man es ohne Zusätze von Katalysatoren, Basen und organischen Lösungsmitteln durchführt.

Das nach der Durchführung der erfindungsgemäßen Decarboxylierung vorliegende Reaktionsgemisch kann man z.B. aufarbeiten, indem man es abkühlt, entspannt, abfiltriert und den abfiltrierten Feststoff mit Wasser wäscht und trocknet. Man erhält so im allgemeinen über 97 % reine Produkte.

Im Hinblick auf den eingangs geschilderten Stand der Technik ist es beim erfindungsgemäßen Verfahren ausgesprochen überraschend, dass es ohne Zusätze von Basen und ohne organische Lösungsmittel gelingt, mit guten Ausbeuten aus 2,3,5,6-Tetrafluorterephthalsäure selektiv eine Carboxylgruppe abzuspalten und das Decarboxylierungsprodukt in hohen Reinheiten zu erhalten. Insbesondere bei Gegenwart einer weiteren Säure kann man besonders selektiv arbeiten.

2,3,5,6-Tetrafluorbenzoesäure ist ein wertvolles Zwischenprodukt zur Herstellung von Pflanzenschutz- und Pharmawirkstoffen.

### Beispiele

### Beispiel 1

In einen 0,6 1 Email-Autoklaven wurden 80 g 80 gew.-%ige 2,3,5,6-Tetrafluorterephthalsäure (enthaltend 8 Gew.-% Schwefelsäure und 12 Gew.-% Wasser) mit 300 g Wasser vermischt und 40 Stunden auf 130°C erhitzt. Nach dem Abkühlen auf 20°C und Entspannen wurde die dann vorliegende Suspension filtriert, mit 60 g Wasser gewaschen und der weiße Feststoff getrocknet. Es wurden 42 g 2,3,5,6-Tetrafluorbenzoesäure mit einem Gehalt von 98 Gew.-% und einen Schmelzpunkt von 151°C erhalten.

### Beispiel 2

Es wurde verfahren wie bei Beispiel 1, jedoch wurden statt 300 g Wasser nur 200 g Wasser hinzugefügt und 6 Stunden auf 140°C erhitzt. Es wurden 47 g 2,3,5,6-Tetrafluorbenzoesäure mit einem Gehalt von 99,4 Gew.-% erhalten.

### Beispiel 3

In einem Email-Autoklaven wurden 100 g 2,3,5,6-Tetrafluorterephthalodinitril mit 140 ml 70 gew.-%iger Schwefelsäure vermischt und 6 Stunden auf 150°C erhitzt. Anschließend wurden 260 ml Wasser zugesetzt und 8 Stunden auf 140°C erhitzt. Nach der wie in Beispiel 1 angegebenen Aufarbeitung des Reaktionsgemisches wurden 78,5 g 99,5 gew.-%ige 2,3,5,6-Tetrafluorbenzoesäure erhalten.

### Beispiel 4

Es wurde verfahren wie in Beispiel 3, jedoch wurden 246 g 70 gew.-%ige Schwefelsäure und zusätzlich 50 g Eisessig eingesetzt, dieses Gemisch 5 Stunden auf 170°C erhitzt, dann 440 ml Wasser zugefügt und 10 Stunden auf 160°C erhitzt. Nach einer Aufarbeitung analog Beispiel 1 wurden 96 g 99,4 gew.-%ige 2,3,5,6-Tetrafluorbenzoesäure erhalten.

### Beispiel 5 (zum Vergleich - siehe JP 93-190 758)

20 g 99,8 gew.-%ige 2,3,5,6-Tetrafluorterephthalsäure, die keine Schwefelsäure enthielt, wurde mit 60 g Wasser 5 Stunden auf 190°C erhitzt. Nach dem Abkühlen bildeten sich im Autoklaven eine organische und eine wäßrige Phase aus. Die organische Phase enthielt 62 % der Theorie 1,2,4,5-Tetraflurobenzol. Die wäßrige Phase enthielt nur Spuren von 2,3,5,6-Tetrafluorbenzoesäure.

### Beispiel 6

In einen Email-Autoklaven von 1,3 1 Inhalt wurden 750 ml Wasser und 80 g 2,3,5,6-Tetrafluorterephthalsäure bei Raumtemperatur vorgelegt. Dann wurde der Autoklav verschlossen und 21 Stunden lang auf 130°C erhitzt, wobei der Druck auf maximal 11,7 bar anstieg. Das mittels HPLC-Analyse untersuchte Reaktionsgemisch enthielt 2,3,5,6-Tetrafluorbenzoesäure in einer Menge, die 78,6 % der Theorie entsprach.

## Patentansprüche

1. Verfahren zur Decarboxylierung von 2,3,5,6-Tetrafluorterephthalsäure unter Bildung von 2,3,5,6-Tetrafluorbenzoesäure, **dadurch gekennzeichnet, dass** man 2,3,5,6-Tetrafluorterephthalsäure zusammen mit Wasser auf Temperaturen von 80 bis 180°C erhitzt

2. Verfahren zur Decarboxylierung von 2,3,5,6-Tetrafluorterephthalsäure unter Bildung von 2,3,5,6-Tetrafluorbenzoesäure, **dadurch gekennzeichnet, dass** man 2,3,5,6-Tetrafluorterephthalsäure zusammen mit Wasser und einer weiteren Säure auf Temperaturen über 80°C erhitzt.

3. Verfahren nach Ansprüchen 1 bis 2, **dadurch gekennzeichnet, dass** man 2,3,5,6-Tetrafluorterephthalsäure in wasserhaltiger Form einsetzt.

4. Verfahren nach Anspruch 2, **dadurch gekennzeichnet, dass** man die 2,3,5,6-Tetrafluorterephthalsäure in noch weitere Säuren enthaltender Form einsetzt.

5. Verfahren nach Ansprüchen 2 und 4, **dadurch gekennzeichnet, dass** man die 2,3,5,6-Tetrafluorterephthalsäure in Form des Reaktionsgemisches einsetzt, das bei der sauren Verseifung des entsprechenden Nitrils anfällt.

6. Verfahren nach Ansprüchen 1 bis 5, **dadurch gekennzeichnet, dass** man es in Gegenwart der 0,5- bis 15-fache Gewichtsmenge Wasser, bezogen auf die eingesetzte 2,3,5,6-Tetrafluorterephthalsäure durchführt.

7. Verfahren nach Ansprüchen 1, 3 und 6, **dadurch gekennzeichnet, dass** man die 2,3,5,6-Tetrafluorterephthalsäure zusammen mit Wasser auf Temperaturen von 90 bis 170°C erhitzt.

8. Verfahren nach Ansprüchen 2, 4 und 6, **dadurch gekennzeichnet, dass** man 2,3,5,6-Tetrafluorterephthalsäure zusammen mit Wasser und einer weiteren Säure auf Temperaturen von 80 bis 200°C erhitzt.

9. Verfahren nach Ansprüchen 1 bis 8, **dadurch gekennzeichnet, dass** man es ohne Zusätze von Katalysatoren, Basen und organischen Lösungsmittels durchführt.

10. Verfahren nach Ansprüchen 1 bis 9, **dadurch gekennzeichnet, dass** man das nach der Decarboxylierung vorliegende Reaktionsgemisch aufarbeitet, indem man es abkühlt, entspannt, abfiltriert und den abfiltrierten Feststoff mit Wasser wäscht und trocknet.

## Claims

1. Process for the decarboxylation of 2,3,5,6-tetrafluoroterephthalic acid with the formation of 2,3,5,6-tetrafluorobenzoic acid, **characterized in that** 2,3,5,6-tetrafluoroterephthalic acid is heated together with water to temperatures of 80 to 180°C.

2. Process for the decarboxylation of 2,3,5,6-tetrafluoroterephthalic acid with the formation of 2,3,5,6-tetrafluorobenzoic acid, **characterized in that** 2,3,5,6-tetrafluoroterephthalic acid is heated together with water and another acid to temperatures above 80°C.

3. Process according to Claim 1 or 2, **characterized in that** 2,3,5,6-tetrafluoroterephthalic acid is used in water-containing form.

4. Process according to Claim 2,**characterized in that** 2,3,5,6-tetrafluoroterephthalic acid is used in a form containing yet other acids.

5. Process according to Claims 2 and 4, **characterized in that** 2,3,5,6-tetrafluoroterephthalic acid is used in the form of the reaction mixture which arises in the acidic saponification of the corresponding nitrile.

6. Process according to Claims 1 to 5, **characterized in that** it is carried out in the presence of 0.5 to 15 times the amount by weight of water, based on the 2,3,5,6-tetrafluoroterephthalic acid used.

7. Process according to Claims 1, 3 and 6, **characterized in that** 2,3,5,6-tetrafluoroterephthalic acid is heated together with water to temperatures of 90 to 170°C.

8. Process according to Claims 2, 4 and 6, **characterized in that** 2,3,5,6-tetrafluoroteraphthalic acid is heated together with water and another acid to temperatures of 80 to 200°C.

9. Process according to Claims 1 to 8, **characterized in that** it is carried out without addition of catalysts, bases and organic solvents.

10. Process according to Claims 1 to 9, **characterized in that** the reaction mixture present after the decarboxylation is worked up by cooling it, depressurizing it, filtering it and washing with water and drying the solid filtered off.

## Revendications

1. Procédé de décarboxylation de l'acide 2,3,5,6-tétrafluorotéréphtalique pour former l'acide 2,3,5,6-tétrafluorobenzoïque, **caractérisé en ce que** l'on chauffe l'acide 2,3,5,6-tétrafluorotéréphtalique avec de l'eau à une température située dans l'intervalle allant de 80 à 180°C.

2. Procédé de décarboxylation de l'acide 2,3,5,6-tétrafluorotéréphtalique pour former l'acide 2,3,5,6-tétrafluorobenzoïque, **caractérisé en ce que** l'on chauffe l'acide 2,3,5,6-tétrafluorotéréphtalique avec de l'eau et un autre acide à une température supérieure à 80°C.

3. Procédé suivant les revendications 1 à 2, **caractérisé en ce que** l'on met en oeuvre l'acide 2,3,5,6-tétrafluorotéréphtalique sous une forme contenant de l'eau.

4. Procédé suivant la revendication 2, **caractérisé en ce que** l'on met en oeuvre l'acide 2,3,5,6-tétrafluorotéréphtalique sous une forme contenant un autre acide.

5. Procédé suivant les revendications 2 et 4, **caractérisé en ce que** l'on met en oeuvre l'acide 2,3,5,6-tétrafluorotéréphtalique sous la forme du mélange réactionnel qui est produit lors de la saponification acide du nitrile correspondant.

6. Procédé suivant les revendications 1 à 5, **caractérisé en ce qu'**on le réalise en présence de 0,5 à 1,5 fois la quantité en poids d'eau, sur base de l'acide 2,3,5,6-tétrafluorotéréphtalique mis en oeuvre.

7. Procédé suivant les revendications 1, 3 et 6, **caractérisé en ce qu'**on chauffe l'acide 2,3,5,6-tétrafluorotéréphtalique avec de l'eau à des températures situées dans l'intervalle allant de 90 à 170°C.

8. Procédé suivant les revendications 2, 4 et 6, **caractérisé en ce qu'**on chauffe l'acide 2,3,5,6-tétrafluorotéréphtalique avec de l'eau et un autre acide à des températures situées dans l'intervalle allant de 80 à 200°C.

9. Procédé suivant les revendications 1 à 8, **caractérisé en ce qu'**on le réalise sans addition de catalyseur, de base et de solvant organique.

10. Procédé suivant les revendications 1 à 9, **caractérisé en ce qu'**on traite le mélange réactionnel présent après la décarboxylation **en ce qu'**on le refroidit, détend, filtre et lave avec de l'eau puis sèche le solide filtré.
